(19) Europäisches Patentamt
European Patent Office
Office européen des brevets

(11) EP 0 517 890 B1

(12) **EUROPEAN PATENT SPECIFICATION**

(45) Date of publication and mention of the grant of the patent:
**02.04.1997 Bulletin 1997/14**

(21) Application number: **92903014.6**

(22) Date of filing: **27.12.1991**

(51) Int. Cl.[6]: **A61L 29/00**, C08J 7/04

(86) International application number:
**PCT/US91/09531**

(87) International publication number:
**WO 92/11877 (23.07.1992 Gazette 1992/19)**

(54) **BIOCOMPATIBLE ABRASION RESISTANT COATED SUBSTRATES**
BIOVERTRÄGLICHE VERSCHLEISSFESTE BESCHICHTETE SUBSTRATE
SUBSTRATS POURVUS D'UN REVETEMENT RESISTANT A L'ABRASION ET BIOCOMPATIBLE

(84) Designated Contracting States:
**AT BE CH DE DK ES FR GB GR IT LI LU NL SE**

(30) Priority: **28.12.1990 US 635914**

(43) Date of publication of application:
**16.12.1992 Bulletin 1992/51**

(73) Proprietor: **UNION CARBIDE CHEMICALS & PLASTICS TECHNOLOGY CORPORATION**
**Danbury, Connecticut 06817-0001 (US)**

(72) Inventors:
- **FAN, You-Ling**
  **East Brunswick, NJ 08816 (US)**
- **MARLIN, Lawrence**
  **Bridgewater, NJ 08807 (US)**

(74) Representative: **Barz, Peter, Dr. et al**
**Patentanwalt**
**Kaiserplatz 2**
**80803 München (DE)**

(56) References cited:
**EP-A- 0 093 094**
**EP-A- 0 106 004**
**EP-A- 0 217 771**
**EP-A- 0 379 156**
**US-A- 4 055 682**
**US-A- 4 729 914**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 517 890 B1

Printed by Rank Xerox (UK) Business Services
2.14.1/3.4

## Description

This invention relates to coating various substrates with poly(carboxylic acids) and, subsequently, poly(ethylene oxide) and/or poly(N-vinyl pyrrolidone). The coatings are biocompatible, abrasion resistant and hydrophilic. The coated substrates of the invention can be used with particular advantage in the medical device, personal care and health care fields.

For many applications, such as long-term in-dwelling catheters, needles and other medical devices, it is desirable to provide a biocompatible hydrophilic coating to an object which is to be inserted into the body. The coating must also be firmly attached to the substrate such that the coating exhibits good abrasion resistance.

Of the many known hydrophilic coatings, the biocompatibility of poly(N-vinyl lactams), poly(carboxylic acids) and poly(alkylene oxides) such as poly(ethylene oxide) ("PEO"), is well known in the art. All of these polymers have been used in a variety of applications where biocompatibility is required. For example, the biocompatibility of a well known poly(N-vinyl lactam), poly(N-vinyl pyrrolidone) ("PVP") is mentioned in the article by S.C. Johnson, "Acetylene Derived Polymers", Cosmetics and Toiletries, Vol. 99, pp. 71-84 (June 1984). Poly(ethylene oxide) either in the form of a homopolymer or copolymer has been widely studied and used for medical applications where excellent biocompatibility with animals or humans is required. The biocompatibility of poly(ethylene oxide) with blood has been studied and reported in several journals; see for example, the article by George, J.J., "Direct Assessment of Platelet Adhesion to Glass", Blood, Vol. 40, p. 862 (1972).

Various methods have been disclosed for adhering poly(carboxylic acids), PVP or PEO coatings to substrates. For example, US-A-4,589,870 discloses a method of applying PVP to a substrate in a solvent blend. The coating renders the substrate hydrophilic.

Another such method is described in US-A-4,642,267 which provides a hydrophilic polymer blend. The blend is comprised of a thermoplastic polyurethane having no reactive isocyanate groups and a hydrophilic poly(N-vinyl lactam) such as PVP. The blend can also contain additional polymeric components such as homopolymers or copolymers of monomers such as vinyl chloride, acrylic acid and vinyl alcohol.

A further method for coating a polymer surface with a hydrophilic coating is disclosed in US-A-4,666,437. The process comprises applying a solution of a compound containing at least two unreacted isocyanate groups per molecule to the polymer surface, and evaporating the solvent. Thereafter, a solution of PVP is applied to the treated surface and the coating is cured.

PCT application WO 89/09246 discloses structures coated with crosslinked hydrophilic polymers. The structure is first coated with a solution of the hydrophilic polymer, such as PVP, and then the structure is dried. The coating is then crosslinked using a thermally-activated or ultraviolet light-activated free radical initiator or alternatively by irradiating the coated surface. The disclosure notes on page 6 lines 22-24, "that [a] hydrophilic polymer crosslinked in the presence of water may produce an adhesive surface rather than a surface having a low coefficient of friction."

EP-A-106004 describes a method of forming a hydrophilic coating on a substrate, said method comprising the application of a hydrophilic copolymer capable of chemically reacting with a polyisocyanate coupling agent previously applied to the surface of said substrate.

US-A-4729914 teaches that an article having a lower coefficient of friction in the wet state can be produced by applying to a substrate an adherent isocyanate coating followed by a second coating of PVP copolymerized with a minor amount of an ethylenic monomer having active hydrogens and curing the so-coated substrate.

EP-A-379156 is directed to a method of covering at least a portion of a substrate with a hydrophilic coating having good abrasion resistance which differs from the method of the present invention as set forth below essentially in that step (3) thereof is omitted.

EP-A-93094 describes a process for the coating of a polymer surface with a hydrophilic coating with low friction in wet condition. The process essentially comprises the application of a polyisocyanate and, thereafter the application of a PEO onto said surface and the subsequent curing at elevated temperatures.

Notwithstanding the teachings of the above references a need exists to provide a more abrasion resistant biocompatible coating on substrates.

The present invention provides abrasion resistant, hydrophilic, biocompatible polymer-coated substrates which can be advantageously used in the medical device, personal care and health care fields. The present invention also provides a method for preparing coated substrates.

The method for preparing the coated substrates comprises the steps of:

(1) contacting said substrate with a polyisocyanate compound contained in an inert solvent to provide an at least partially coated substrate;

(2) contacting said at least partially coated substrate with a poly(carboxylic acid), said poly(carboxylic acid) contained in an inert solvent, to provide a multiple coated substrate;

(3) contacting said multiple coated substrate with an inert solvent containing at least one polymeric compound selected from a poly(N-vinyl pyrrolidone) and a poly(ethylene oxide);

(4) curing said multiple coated substrate by means of ionizing radiation or heat or both.

Previously, high molecular weight poly(alkylene oxides), poly(carboxylic acids) and poly(N-vinyl lactams) were difficult to bond to the surface of many materials to provide a coated article having high abrasion resistance. This shortcoming is overcome by the method of the present invention by coating biocompatible polymers on the isocyanate precoat and curing the coating by drying or radiation or both. When the coatings are cured by drying, poly(carboxylic acid) is reacted with the isocyanate and also complexed with PEO or PVP or both. The resulting biocompatible polymeric complex is securely bonded to the substrate and is abrasion resistant.

Curing the polymers by irradiation results in a cross-linked polymeric coating. The cross-linked coating provided by the irradiation of the polymers is very abrasion resistant. The use of ionizing radiation to cure the coating is the preferred embodiment of the present invention.

Cross-linking of a hydrophilic polymer may lead to a loss of surface lubricity due to the loss of mobility of the macromolecules on the surface. However, by employing the method of the present invention the limited surface macromolecules not only form a cross-linked network which improves the abrasion resistance of the coating, but at the same time can provide a lubricious surface depending on the radiation dose. Consequently a medical device, such as a catheter, coated in accordance with the method of this invention can retain a biocompatible surface for long periods of time.

Application of the polyisocyanate solution and the poly(carboxylic acid), PEO and PVP solutions can be effected by a variety of methods which include, but are not limited to, dipping, spraying, electrical deposition, painting and the like. Dip coating of the substrate in the respective solutions is the preferred method. It is to be understood that the above solutions also include respective dispersions and emulsions of the polyisocyanate and polymers.

A wide variety of polyisocyanates can be employed in preparing the coatings of the present invention and include, but are not limited to, toluene-2,4-diisocyanate, toluene-2,6-diisocyanate, commercial mixtures of toluene-2,4- and 2,6-diisocyanates, 4,4'-diphenylmethane diisocyanate, cyclohexylene-1,4-diisocyanate, m-phenylene diisocyanate, 3,3-diphenyl-4-biphenylene diisocyanate, 4,4-biphenyl diisocyanate, 1,6-hexamethylene diisocyanate, 1,5-naphthalene diisocyanate, cumene-2,3-diisocyanate, 2,4-diisocyanatodiphenylether, isocyanate end-capped prepolymers and adducts, isocyanate end-capped polyfunctional aromatic adducts, isocyanate end-capped polyfunctional aliphatic adducts, and two component systems such as end-capped aliphatic polyester polyols and their mixtures with different polyisocyanates as described above.

Illustrative of isocyanate end-capped adducts are the reaction products of toluene-2,4-diisocyanate, 4,4'-diphenylmethane diisocyanate, polymethylenepolyphenyl isocyanate, or 1,5-naphthalene diisocyanate, with 1,2-polypropylene glycol, polytetramethylene ether glycol, 1,4-butanediol, 1,3-butanediol, poly(1,4-oxybutylene) glycol, caprolactone, adipic acid esters, phthalic anhydride, ethylene glycol, diethylene glycol, and the like.

Suitable solvents for applying the polyisocyanate precoat to the substrate in the first step of the process of this invention include: methyl ethyl ketone, ethyl acetate, ethyl lactate, chloroform, trichloroethylene, dichloromethane, hexane, heptane, toluene, and mixtures thereof, which do not react with isocyanates under the coating conditions. The preferred solvents are toluene and methyl ethyl ketone.

Alternatively, the polyisocyanates can be either dispersed in a solvent/non-solvent mixture to form a dispersion or emulsified to form an oil-in-water emulsion. When an emulsion is used, the reactive isocyanate groups need to be protected by suitable chemical groups known to those skilled in the art.

Illustrative poly(ethylene oxide) polymers suitable for the purpose of this invention are represented by the following formula:

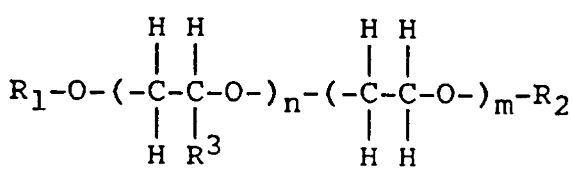

wherein: $R_1$ and $R_2$ are hydrogen, alkyl groups or alkyl-substituted aryl groups; $R^3$ is an alkyl group having 1 to 8 carbons, $R^3$ preferably being methyl; n is an integer from 0 to 20,000; and m is an integer from about 2500 to about 180,000, provided that the values of n and m are such that the poly(ethylene oxide) is water-soluble or, at least, water swellable.

Examples of high molecular weight poly(ethylene oxides) suitable for use in this invention include the POLYOX® Water Soluble Resins, such as POLYOX® WSR N-80, WSR N-750, WSR N-3000, WSR N-60K, WSR-301, and Coag. grade, available from Union Carbide Chemicals and Plastics Company Inc., Danbury, CT.

The poly(N-vinyl pyrrolidones) suitable for the purpose of this invention, have a molecular weight between about 100,000 and about 3,000,000. Such polymers are readily produced by solution polymerization of N-vinyl pyrrolidone in the presence of a suitable free radical initiator. Examples of high molecular weight PVP include Plasdone® K-90 and PVP K-60, both available from GAF Corporation.

Another class of polymers which is used in this invention are the poly(carboxylic acids). They are useful in either their free acid or partially neutralized acid forms. Preferred poly(carboxylic acids) are those encompassed by the following formula which includes both the free acid and partially neutralized forms:

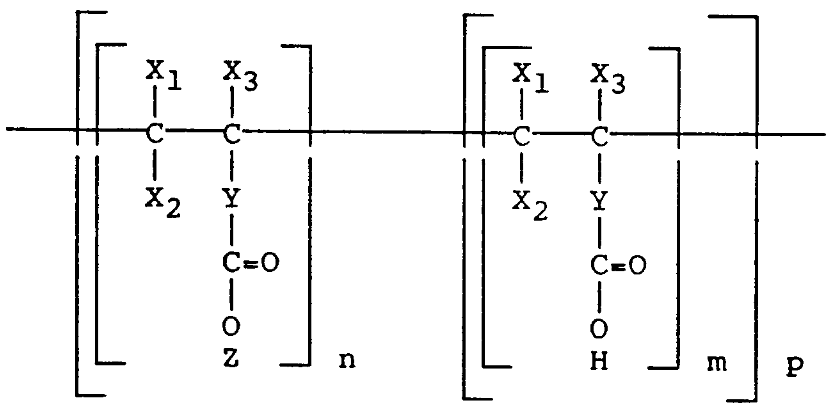

where:

n = 0-0.95 mole fraction of neutralized acid moieties;
m = 0.05-1.0 mole fraction of acid moieties with the proviso that n+m = 1 ;
$X_1$, $X_2$, $X_3$ are each a hydrogen atom or a suitable monovalent organic group, such as lower ($C_1$-$C_4$) alkyl groups or cycloalkyl or aryl groups of up to 8 carbon atoms, and wherein the X groups are such that the polymer is water soluble;
Y is either a single bond or any suitable divalent organic radical, such as a hydrocarbon group of up to 8 carbon atoms, provided it does not adversely affect the water solubility of the polymer;
Z is either a metallic ion such as any of the alkali metal cations or a suitable ammonium ion; and
p is a very large number such that the polymer has a molecular weight between about 200,000 and about 5,000,000.

Even though all poly(carboxylic acid) homopolymers are useful in varying degrees, the high molecular weight polymers are more desirable for use in this invention. The more useful are those having molecular weights within the range from about 200,000 to about 5,000,000. Especially useful are the poly(carboxylic acid) polymers having a molecular weight from about 1,000,000 to about 3,000,000.

Representative carboxylic acid-containing homopolymers include, but are not limited to, poly(acrylic acid), poly(methacrylic acid), poly(isocrotonic acid), and the like. The poly(carboxylic acid) of this invention can be either a solution or a colloidal dispersion in the coating medium. The preferred poly(carboxylic acid) polymer is a poly(acrylic acid) having a molecular weight of from about 200,000 to about 5,000,000. Particularly preferred poly(carboxylic acids) are those having a molecular weight from about 1,000,000 to about 3,000,000.

Also suitable for use in this invention are poly(carboxylic acid) polymers containing comonomer units. Such polymers are produced by copolymerizing an olefinic acid such as acrylic with one or more other ethylenically unsaturated monomers to produce copolymers containing carboxylic acid moieties. Examples of such copolymers include Carboset® and Surlyn® available from by B.F. Goodrich Chemical Co. and E.I. DuPont de Nemours and Company, Inc. respectively. Copolymers containing water-insoluble units as well as carboxylic acid units can be mixed with the homopolymers if so desired, as long as they are biocompatible.

To prepare a seed-free poly(carboxylic acid) solution, it is advantageous to add a small amount of surfactant in a solvent before mixing with the polymer. Any soluble surfactant or a mixture of surfactants in suitable solvents such as dimethyl formamide and acetonitrile may be used. The preferred surfactants are soluble non-ionic surfactants such as polyoxyethylene sorbitan fatty acid esters, polyoxyethylene acids, polyoxyethylene alcohols, fluorinated alkyl esters, fluorinated alkoxylates and their mixtures.

Due to the high molecular weight of the poly(carboxylic acid) polymers preferred for use in the present invention, their solution viscosities may be too high to be suitable for some coating processes. It is advantageous in these

instances to convert the polymer solution to a colloidal dispersion by mixing with one or more non-solvents. Examples of such non-solvents are tertiary alcohols, ketones, aliphatic ethers, and aliphatic and aromatic hydrocarbons. The preferred non-solvents are acetone, methyl ethyl ketone (MEK) and tertiary butyl alcohol.

Alternatively, the poly(carboxylic acid) may be emulsified to a water-in-oil emulsion. An example for forming such a water-in-oil emulsion is described in US-A-4,618,647.

If desired, one can use other oxygen-containing polymeric compounds in combination with the PVP, poly(carboxylic acid) and PEO as long as such other compounds are biocompatible. Such compounds include nonionic, amphoteric, anionic or cationic polymers such as methyl cellulose, carboxymethylcellulose, sodium carboxymethylcellulose, hydroxyethylcellulose, quaternized hydroxyethyl cellulose, polyvinyl alcohol, maleic anhydride-vinyl ether copolymers, ethylene-maleic anhydride copolymers, dextran, gelatin, hydroxypropylcellulose, hydroxyethylcarboxymethyl-cellulose, propylene glycol alginate, chitosan and derivatives, hyaluronic acid and its salts, and the like.

Other additives can be employed in the coatings such as, stabilizers, surfactants, antioxidants, antimicrobial agents, colorants, biological components and the like. For example, in catheters which are inserted into blood vessels, it may be desirable to have contained in the coating an antithrombogenic agent such as heparin, to avoid blood clot formation during the surgical procedure. The antithrombogenic agent can be used either as an additive or as a chemically bonded moiety of the final coating.

Antimicrobial agents which can be either used as an additive or as a chemically bonded moiety of the coating include silver nitrate, chlorhexidine gluconate or chlorhexidine acetate. Similarly pharmaceutical or therapeutic agents described in US-A-4,946,870 may also be advantageously used in the present invention.

In practice it has been found that excellent adhesion and abrasion resistance properties are obtained when the total thickness of the isocyanate coating and top coating applied to the substrates in accordance with the teachings of this invention is from the sub-µm range to below 100 µm, preferably below ten µm.

The concentration of the isocyanate can vary depending upon the particular components employed, their solubility as well as other considerations. In general, the polyisocyanate concentration in the initial coating is at least about 0.01% by weight. It is preferred to employ the polyisocyanate in a solvent in a concentration of from about 0.1 to about 20% by weight, and more preferably from about 1 to about 5% by weight.

In practice the poly(carboxylic acid), PEO and PVP coatings are usually applied in a thickness of from about 0.1 to about 50 µm and more preferably from about 0.5 to about 10 µm. The solvent system for application of the coatings is one which does not adversely effect the durability of the final coating and can include both organic and aqueous systems. Illustrative compounds which can be utilized as a solvent for the final coating include water and organic solvents such as, methylene chloride, 1,1-dichloroethane, 2,2-dichloroethane, 1,1,1-trichloroethane, 1,1,2-trichloroethane, 1,1,2,2-tetrachloroethane, toluene, benzene, chloroform, cyclohexane, pyridine, acetone, nitromethane and the like.

In general the polymeric compounds comprising the poly(carboxylic acid), PVP and/or PEO are contained in a solvent in a concentration of from about 0.01 to about 10 weight percent and more preferably from about 0.1 to about 2 weight percent based on the total weight of solution.

The drying temperatures and times are not necessarily critical. It has been found that the coated substrate can be dried at temperatures of from about 20 to about 150°C and more preferably from about 50 to about 95°C. Drying periods can range from a few seconds to 24 hours or more.

Although not required, the coatings can be dried prior to the application of the subsequent coating or prior to exposure to the radiation source. In a preferred embodiment the substrate is oven dried after the initial isocyanate coating and after the subsequent layers are applied, before the substrate is irradiated.

The suitable sources of ionizing radiation used for the irradiation of the coated substrates include gamma rays, α-particles, β-particles, cobalt-60, and an electron beam. The preferred ionizing-radiation source is an electron beam generated by a Van De Graff generator.

The irradiation dosage suitable for the purpose of this invention may vary over a broad range depending on the intended end-use application of the coated substrate. At the low end, a dosage is needed to transform the coating to a gel-like consistency. At the high end, the dosage must be controlled such that the coating does not become brittle or degraded. A durable and lubricious coating can be achieved by intermediate radiation dosages. The preferred dosage ranges between about 0.5 to about 5 mrad.

Water may be present during the irradiation. In a preferred embodiment, the substrate is irradiated in the presence of water. Water may be incorporated into the coating either before or during the irradiation by any convenient method such as dipping, spraying, or brushing. While a wide range of water content in the coating may be used, it is desirable that sufficient water in the coating be maintained such that a gel-like consistency is achieved upon irradiation. It has been surprisingly discovered that when the substrate is irradiated either in the presence or absence of water, the surface can be advantageously employed for coating medical devices. The teaching of the prior art discloses that irradiation in the presence of water provides tacky, undesirable surfaces.

Accordingly, the method of the present invention can be used for the coating of medical devices, where a slippery exterior and/or interiors are necessary or desirable to minimize injury to tissues and to aid in manipulation of the devices during surgical procedures.

Illustrative medical devices that can be coated by the method of the invention are catheters, needles, guide wires, prophylactic devices, tubing, delivery systems, filters, sheaths, and other accessories employed in medical diagnostics, drainage, dilation, occlusion, oncology, ophthalmology, orthopedics, reconstructive surgery, anesthesiology, dermatology, dental, ENT, vascularport infusion devices, phlebotomy, critical care vascular access, and the like.

Since the coating provides a biocompatible surface it is possible to entirely coat a substrate which itself is not biocompatible and therefore utilize the desirable features of the substrate material which might not otherwise be biocompatible. For example, the fabrication of prosthetic devices, catheters, guide wires and the like used in the medical field has largely been limited to materials which are known to be inert and which do not affect the body. The method of the present invention renders materials which are not biocompatible, suitable for use in medical devices and other related applications since body tissue is in contact with a biocompatible coating.

Accordingly, substrates such as polyacrylates, nylon, polypropylene, silicone rubbers, thermoplastic rubbers (such as butadiene-styrene copolymers), polyesters (such as Dacron®), stainless steel, cobalt-chromium alloys, platinum, polyethylene, pyrolytic carbon disks or balls, segmented polyurethanes, alumina, polysulfone and the like, can be employed as the substrates.

The Examples which follow are presented for the purpose of illustrating the invention and are not to be construed as unduly limiting the claims. All parts and percentages are by weight unless otherwise specified.

Polyisocyanate Solution A: This polyisocyanate solution was prepared by mixing two reaction adducts with toluene.

The first adduct was prepared by mixing 4,4'-diphenylmethane diisocyanate and a polyether polyol to produce an adduct containing 23.5% by weight active isocyanate, an equivalent weight of 182 and a viscosity of 770 mPa • s(centipoise) at 25°C. The second adduct was prepared by reacting dicyclohexylmethane-4,4-diisocyanate and a polyether polyol to produce an adduct containing 12.1% by weight active isocyanate having a viscosity of 500-1500 mPa • s (centipoise) at 25°C (measured as a 60% solution in toluene).

Polyisocyanate Solution A was prepared by mixing 0.9 part of the first adduct with 1.02 parts of the second adduct and 98.08 parts by weight toluene.

Polyisocyanate Solution B: This polyisocyanate solution was prepred by mixing 83.2 parts of methyl ethyl ketone, 15 parts of mineral oil and 1.8 parts of polyisocyanate. The polyisocyanate employed was an aromatic isocyanate end-capped prepolymer, average NCO equivalent weight 182. The resulting clear liquid contained 0.42% by weight isocyanate groups.

Polyisocyanate Solution C: This polyisocyanate solution was prepared by mixing 98.2 parts of methyl ethyl ketone and 1.8 parts of the polyisocyanate employed in making Polyisocyanate Solution B. The solution contained 0.42% by weight isocyanate groups.

PEO Solution A in Water: A 2% PEO solution in water was prepared by gentle mixing of 10 gms of POLYOX® Coagulant grade (molecular weight of 5,000,000) in 490 gms of distilled water at room temperature. The finished solution possessed a Brookfield viscosity of 5630 mPa • s(cps) (Model LVT, 30 rpm, at 25°C).

PEO Solution B in Water: A 3% PEO solution in water was prepared by gentle mixing of 15 gms of POLYOX® Coagulant grade in 485 gms of distilled water at room temperature. The finished solution possessed a Brookfield viscosity of 12,600 mPa • s(cps)(Model LVT, 30 rpm, at 25°C).

PEO Solution C in Dichloromethane: A 1.5% PEO solution in dichloromethane was prepared by gentle mixing of 7.5 gms of POLYOX® Coagulant grade in 492.5 gms of dichloromethane. The finished solution was clear and had a Brookfield viscosity below 100 mPa • s(cps) (Model LVT, 50 rpm, at 25°C).

Poly(acrylic acid) Dispersion: This dispersion is a high molecular weight poly(acrylic acid) dispersion available commercially from Union Carbide Chemicals and Plastics Company Inc., Danbury, Ct., as POLYSLIP® T-503M.

## Example 1

An 8 French catheter was cut to 30 cm (12 inches) in length, wiped with Freon® and air dried for 5 minutes. The catheter was then dipped in Polyisocyanate Solution A for 30 seconds and air dried for 1 minute, followed by drying in a forced air oven for 30 minutes at 50°C. Thereafter, the catheter was dipped in Poly(acrylic acid) Dispersion (i.e.,POLYSLIP® T-503M) for one second and allowed to air dry for one minute before being dried in a forced air oven for 60 minutes at 50°C The catheter was then dipped in PEO Solution A for one second and allowed to air dry for 1 minute and then dried in a forced air oven at 50°C for 10-1/2 hours. The dried catheter was dipped in a distilled water bath for 10 seconds, sealed in a polyethylene bag, and irradiated under an electron-beam source for 1.5 mrad. The catheter was

lubricious, non-sticky, and was readily removed from the polyethylene bag.

Comparative Example A

An 8 French catheter was cut to 30 cm (12 inches) in length, wiped with Freon® and air dried for 5 minutes. The catheter was then dipped in PEO Solution A for one second, air dried for one minute and then dried in a forced air oven for 10-1/2 hours. The dried catheter was dipped in a distilled water bath for 10 seconds, sealed in a polyethylene bag, and irradiated under an electron-beam source for 1.5 mrad. The catheter's coating was lubricious but sticky and loosely adhered to the catheter.

Example 2

Example 1 was repeated with the exception that the water swollen, coated catheter was irradiated for 2.5 instead of 1.5 mrad. The catheter was lubricious, non-sticky, and was readily removed from the polyethylene bag.

Comparative Example B

Comparative Example A was repeated with the exception that the water swollen, coated catheter was irradiated with 2.5 instead of 1.5 mrad. The catheter was lubricious but the coating exhibited marginal adhesion to the catheter.

Comparative Example C

Comparative Example A was repeated with the exception that the PEO coated catheter was not irradiated. The coating was lubricious but somewhat sticky when wet and loosely adhered.

Example 3

The hydrophilicity, lubricity and abrasion resistance of the coated catheters repared in Example 1, and Comparative Examples A and C were measured and compared. The hydrophilic lubricity was measured using an incline-platform tester as described in EP-A-166,998. The results are expressed as frictional coefficients which are equal to the tan $\theta$ of the measured angles. The smaller the angle, the smaller the frictional coefficient, the better the lubricity. Abrasion resistance of the coating was measured by abrading the wet catheter through a spherical hole punched in a rubbery membrane. The catheter is abraded through the hole ten times. (The diameter of the hole is slightly smaller than the outside diameter of the uncoated catheter). The frictional coefficient of the abraded sample was measured; the results are reported below.

The wet coating removed from the catheter during abrasion was collected and weighed; the results are listed in the last column of Table I. The wet coating collected in this Example was found to contain more than 90% by weight water.

Comparative Example C demonstrates the poor coating quality and a lack of abrasion resistance of PEO coating applied directly from PEO Solution A. Although Comparative Example A illustrates some benefit of irradiating the PEO coating in the presence of water, the abrasion resistance was still unsatisfactory.

Table I

| Example | Radiation Dosage, mrad | Frictional Coeff. Before Abrasion | Frictional Coeff. After Abrasion | Wet Coating Removed by Abrasion, mg* |
|---|---|---|---|---|
| 1 | 1.5 | 0.05 | 0.06 | 3 |
| A | 1.5 | 0.07 | 0.11 | 30 |
| C | - | >0.2 | >0.2 | 146 |

*Per a (12-inch) 30 cm catheter weighing 1.0 gm dry weight.

The above results again demonstrate the advantage of the current invention. The coating prepared in Example 1 exhibits improved abrasion resistance and a high degree of lubricity. The coatings of Comparative Examples A and C demonstrated poor abrasion resistance.

Comparative Example D

Comparative Example A was repeated with the exception that PEO Solution B was substituted for PEO Solution A. The finished coating was lubricious but lacking in abrasion resistance (see Table III below).

Comparative Example E

Comparative Example D was repeated with the exception that a higher radiation dosage was employed (2.5 mrad). The finished coating was lubricious but exhibited unsatisfactory abrasion resistance.

Examples 4-6

These Examples illustrate the performance characteristics of coatings prepared in accordance with the procedure of Example 1, except that different concentrations of Poly(acrylic acid) Dispersion ("PAA") and different radiation dosages were used. The particular concentrations of PAA and radiation dosages used, as well as the results are given in Table II.

Table II

| Example | Concentration of PAA Solution Used, % | Radiation Dosage, mrad | Frictional Coeff. Before Abrasion | Frictional Coeff. After Abrasion | Wet Coating Removed by Abrasion, mg |
|---|---|---|---|---|---|
| 1 | 2 | 1.5 | 0.05 | 0.06 | 3 |
| 4 | 2 | 2.5 | 0.06 | '0.05 | 0 |
| 5 | 3 | 1.5 | 0.05 | 0.05 | <1 |
| 6 | 3 | 2.5 | 0.06 | 0.05 | 0 |

In all cases, the coatings exhibit a combination of good abrasion resistance and high lubricity.

Comparative Example F

The following Examples compare the performance characteristics of irradiated PEO coatings which were coated on substrates without an initial polyisocyanate coating on the substrate. Comparative Example F was done following the procedure of comparative Example A except that a higher radiation dosage was employed.

Table III

| Example | Concentration of PEO Solution Used, % | Radiation Dosage, mrad | Frictional Coeff. Before Abrasion | Frictional Coeff. After Abrasion | Wet Coating Removed by Abrasion, mg |
|---|---|---|---|---|---|
| B | 2 | 1.5 | 0.07 | 0.11 | 30 |
| G | 2 | 2.5 | 0.08 | >0.2 | 204 |
| E | 3 | 1.5 | 0.11 | >0.2 | 237 |
| F | 3 | 2.5 | 0.14 | >0.2 | 207 |

In all cases, the coatings exhibited poor abrasion resistance and poor lubricity.

Comparative Example G, Example 7

The catheters used in Comparative Example G and Example 7 were coated with PEO Solution C using procedures otherwise substantially the same as those described in Comparative Example A and Example 1, respectively. The performance characteristics of the finished coatings are compiled in Table IV.

Table IV

| Example | Concentration of PEO Solution Used, % | Radiation Dosage, mrad | Frictional Coeff. Before Abrasion | Frictional Coeff. After Abrasion | Wet Coating Removed by Abrasion, mg |
|---|---|---|---|---|---|
| G | 1.5 | 1.5 | 0.08 | >0.2 | 15 |
| 7 | 1.5 | 1.5 | 0.05 | 0.05 | 2 |

These results demonstrate the improved performance of illustrative coatings which combine both an isocyanate coating and PEO coating before irradiation. Without the isocyanate coating (Comparative Example G), both the amount of coating removed from the catheter and the frictional coefficient is greatly increased by the abrasion.

Example 8

Four groups of C-Flex catheters (Concept Polymer) were coated with a 1% poly(ethylene oxide) (Union Carbide POLYOX® WSR N-750) solution in either methylene chloride or water.

In all of the trials the surface of the catheters was first prepared by wiping the catheter with a Freon® containing cloth and air drying for five minutes.

In Comparative Trials A and B the PEO solution was applied directly to the catheter and dried in a forced air oven at 60°C for 60 minutes. In Comparative Trial C, Polyisocyanate Solution C was applied to the catheter and dried in an oven at 60°C for 30 minutes prior to the application of the PEO solution. In Trial D, the catheter was dipped in Polyisocyanate Solution B and dried in a forced air oven at 60°C for 30 minutes. The catheter was next dipped in the Poly(acrylic acid) Dispersion and baked in a forced air oven at 60°C for 60 minutes. The catheter was then dipped in the PEO solution for ten seconds and dried in an oven at 60°C for 30 minutes.

The frictional coefficient of the catheters was measured before and after the abrasion test described below.

A coated catheter is rubbed with a piece of wet tissue (Kimwipes, Kimberly Clark) folded to about 5 x 5 (2x2") in size and wrapped around the circumference of the catheter. Finger pressure is applied to the wet wrapping against the catheter surface, and the wrapping is pulled longitudinally from one end to the other. After ten rubs have been made, a pair of the abraded catheters are retested for frictional coefficient according to the procedure described above. Adhesion of the wet coating is judged by the extent of change in frictional coefficient after the abrasion test.

The frictional coefficients for the coated catheters of Example 8 are listed in Table V.

Table V

| Trial | PEO + Other Coating(s) Applied | PEO Solvent | Frictional Coeff. Before Abrasion | Frictional Coeff. After Abrasion |
|---|---|---|---|---|
| A(*) | None | water | >0.2 | >0.2 |
| B(*) | None | methylene chloride | >0.2 | >0.2 |
| C(*) | Polyisocyanate | methylene chloride | 0.12 | >0.2 |
| D | Polyisocyanate and Poly(acrylic acid) | water | 0.06 | 0.06 |

(*) = comparative

The catheters treated in Comparative Trials A and B had a high frictional coefficient before and after the abrasion test. This demonstrates that little or no poly(ethylene oxide) bonded to the surface of the catheters. The catheter treated in Comparative Trial C had a lower frictional coefficient initially but the frictional coefficient increased after the abrasion test. This loss of lubricity demonstrates a lack of adhesion of the poly(ethylene oxide) to the surface. In Trial D, the frictional coefficient was low and remained the same before and after the abrasion test demonstrating that the PEO was securely bonded to the catheter.

Example 9

Respective C-Flex catheters were coated with a 2% poly(vinyl pyrrolidone) (SIGNA PVP-360) solution. The PVP was dissolved in either methylene chloride or water.

Four catheters were coated using the same methods described in Example 8. In Comparative Trials A and B the respective PVP solutions were applied directly to the catheter. In Comparative Trial C, the PVP coating was applied after Polyisocyanate Solution C was applied to the catheter. In Trial D, Polyisocyanate Solution B and the Poly(acrylic acid) Dispersion were applied to the catheter prior to the application of the PVP solution.

The frictional coefficient of the catheters was measured before and after abrasion. The results are listed in Table VI.

Table VI

| Trial | PVP + Other Coating(s) Applied | PVP Solvent | Frictional Coeff. Before Abrasion | Frictional Coeff. After Abrasion |
|---|---|---|---|---|
| A(*) | None | water | >0.2 | >0.2 |
| B(*) | None | methylene chloride | >0.2 | >0.2 |
| C(*) | Polyisocyanate | methylene chloride | >0.2 | >0.2 |
| D | Polyisocyanate and Poly(acrylic acid) | water | 0.1 | 0.12 |

(*) = comparative

The catheters treated in Comparative Trials A, B, and C had a high frictional coefficient before and after the abrasion test. This demonstrates that little or no PVP was bonded to the surface of the catheters. In Trial D the frictional coefficient of the catheter before the abrasion test was significantly lower than that of the other catheters tested. This result demonstrates that PVP was bonded to the catheter. The slight increase in the frictional coefficient after the abrasion test demonstrates that the PVP is firmly bonded to the catheter.

**Claims**

**1.** A method of forming a hydrophilic polymeric coating on a substrate to provide a biocompatible surface having abrasion resistance, said method comprising:

(1) contacting said substrate with a polyisocyanate compound contained in an inert solvent to provide an at least partially coated substrate;

(2) contacting said at least partially coated substrate with a poly(carboxylic acid), said poly(carboxylic acid) contained in an inert solvent, to provide a multiple coated substrate;

(3) contacting said multiple coated substrate with an inert solvent containing at least one polymeric compound selected from a poly(N-vinyl pyrrolidone) and a poly(ethylene oxide);

(4) curing said multiple coated substrate by means of ionizing radiation or heat or both.

**2.** The method of claim 1 wherein said poly(carboxylic acid) is poly(acrylic acid).

**3.** The method of claim 1 wherein the resultant coatings additionally comprise one or more additives selected from stabilizers, surfactants, antioxidants, antimicrobial agents, antithrombogenic agents and colorants.

**4.** A coated substrate having at least a portion thereof coated by the method of claim 1.

**5.** The coated substrate of claim 4 which is a medical device.

**6.** The medical device of claim 5 which is a catheter or a guide wire.

## Patentansprüche

1. Verfahren zur Bildung eines hydrophilen polymeren Überzugs auf einem Substrat, um eine biokompatible Oberfläche mit Abriebbeständigkeit bereitzustellen, umfassend:

   (1) Kontaktieren des Substrats mit einer in einem inerten Lösungsmittel enthaltenen Polyisocyanat-Verbindung, um ein zumindest teilweise beschichtetes Substrat bereitzustellen;
   (2) Kontaktieren des zumindest teilweise beschichteten Substrats mit einer Polycarbonsäure, wobei die Polycarbonsäure in einem inerten Lösungsmittel enthalten ist, um ein mehrfach beschichtetes Substrat bereitzustellen;
   (3) Kontaktieren des mehrfach beschichteten Substrats mit einem inerten Lösungsmittel, das mindestens eine aus einem Poly(N-vinylpyrrolidon) und einem Poly(ethylenoxid) ausgewählte polymere Verbindung enthält;
   (4) Härtung des mehrfach beschichteten Substrats mit Hilfe von ionisierender Strahlung oder Wärme oder beiden.

2. Verfahren nach Anspruch 1, in welchem die Polycarbonsäure Polyacrylsäure ist.

3. Verfahren nach Anspruch 1, in welchem die resultierenden Überzüge zusätzlich ein oder mehrere aus Stabilisatoren, Tensiden, Antioxidationsmitteln, antimikrobiellen Mitteln, antithrombogenen Mitteln und Färbemitteln ausgewählte Additive umfassen.

4. Beschichtetes Substrat, das mindestens einen Teil davon durch das Verfahren nach Anspruch 1 beschichtet aufweist.

5. Beschichtetes Substrat nach Anspruch 4, bei dem es sich um eine medizinische Vorrichtung handelt.

6. Medizinische Vorrichtung nach Anspruch 5, bei der es sich um einen Katheter oder einen Führungsdraht handelt.

## Revendications

1. Procédé de formation d'un revêtement polymérique hydrophile sur un substrat pour produire une surface biocompatible résistant à l'abrasion, ce procédé comprenant :

   (1) la mise en contact du substrat avec un composé polyisocyanate contenu dans un solvant inerte pour obtenir un substrat au moins partiellement revêtu,
   (2) la mise en contact de ce substrat au moins partiellement revêtu avec un poly(acide carboxylique), ce poly(acide carboxylique) étant contenu dans un solvant inerte, pour obtenir un substrat à revêtement multiple,
   (3) la mise en contact de ce substrat à revêtement multiple avec un solvant inerte contenant au moins un composé polymérique choisi parmi une poly(N-vinyl-pyrrolidone) et un poly(éthylène-oxyde),
   (4) le durcissement du substrat à revêtement multiple au moyen de rayonnement ionisant ou de chaleur ou des deux.

2. Procédé selon la revendication 1, dans lequel le poly(acide carboxylique) est un poly(acide acrylique).

3. Procédé selon la revendication 1, dans lequel les revêtements obtenus comprennent en plus un ou plusieurs additifs choisis parmi les stabilisants, agents tensioactifs, antioxydants, agents antimicrobiens, agents antithrombogéniques et colorants.

4. Substrat revêtu ayant au moins une partie revêtue par le procédé de la revendication 1.

5. Substrat revêtu de la revendication 4 qui est un dispositif médical.

6. Dispositif médical de la revendication 5 qui est un cathéter ou un fil-guide.